# EUROPEAN PATENT APPLICATION

(11) **EP 0 712 632 A1**
(43) Date of publication of application: **22.05.1996**
(21) Application number: 95914549.1
(22) Date of filing: 10.04.1995
(51) Int. Cl.: A61K 39/395, C12P 21/08, C07K 16/24

(54) **REMEDY FOR DISEASES CHARACTERIZED BY ABNORMAL ACCUMULATION OR ACTIVATION OF LEUKOCYTE**

(30) Priority: 08.04.1994 JP 70969/94
(71) Applicant: TORAY INDUSTRIES, INC., Tokyo 103 (JP)
(72) Inventor: MATSUSHIMA, Kouji, Ishikawa 921 (JP); MATSUMORI, Akira, Osaka 562 (JP); NARUTO, Masanobu, Kanagawa 248 (JP); HARADA, Kenji, Kamakura-shi Kanagawa 248 (JP); NARUMI, Hideki, Kanagawa 248 (JP); IDA, Nobuo, Tokyo 195 (JP)
(74) Representative: Kador & Partner
(86) International application number: JP9500698
(87) International publication number: WO9527509

(57) **Abstract**

A remedy for diseases characterized by abnormal accumulation and/or activation of leukocytes, containing an antibody inhibiting the monocyte or macrophage chemotaxis toward a pathogenic region as the active ingredient.

## Description

### TECHNICAL FIELD

The present invention relates to a therapeutic agent for preventing or curing diseases characterized by abnormal accumulation or activation of leukocytes.

### BACKGROUND ART

When monocytes and macrophages migrate to a lesion (This phenomenon is also called "chemotaxis". In the present specification hereinbelow, the phenomenon of migration is called "migration", and the property to migrate is called "chemotaxis".), physiologically active substances originated from monocytes and macrophages are liberated, so that the chain of inflammation starts. Although migration of monocytes and macrophages are observed in general inflammation, it is prominently observed in acute serious inflammation, chronic intractable inflammation and allergies, as well as in lung fibrosis, arteriosclerosis, malignant tumors and the like. In lung diseases, the number of macrophages is known to increase in the lung, and macrophages play an important role in lung fibrosis. Accumulation of macrophages is also observed in the lesions of chronic rheumatoid arthritis (RA). It is known that various cytokines released from different tissues play important roles in migration to and invasion into the loci of inflammation reaction. Among the cytokines, monocyte chemotactic and activating factor (hereinafter also referred to as "MCAF") is a factor which attracts and activates monocytes and macrophages.

Against the above-mentioned diseases, nonsteroidal anti-inflammatory drugs are conventionally used. Further, although steroidal anti-inflammatory drugs are known to inhibit migration of monocytes and macrophages, they have a drawback in that they also inhibit functions of various cells, so that they bring about various side effects. No drug is known which is used as therapeutic and prophylactic agent against diseases caused by MCAF, and which selectively inhibits the action of MCAF *in vivo*.

### DISCLOSURE OF THE INVENTION

Accordingly, an object of the present invention is to provide prevention and therapy of diseases characterized by abnormal accumulation and activation of leukocytes, especially monocytes or macrophages, against which there are no effective drugs so far.

That is, the present invention provides therapeutic agents for diseases characterized by abnormal accumulation or activation of leukocytes, comprising as an effective ingredient an antibody which inhibits migration of monocytes and/or macrophages to pathologic lesions.

By the present invention, a therapeutic agent which enables prevention and therapy of diseases characterized by abnormal accumulation and activation of leukocytes, especially monocytes or macrophages, against which there are no effective drugs so far, was first provided.

### BRIEF DESCRIPTION OF THE DRAWINGS

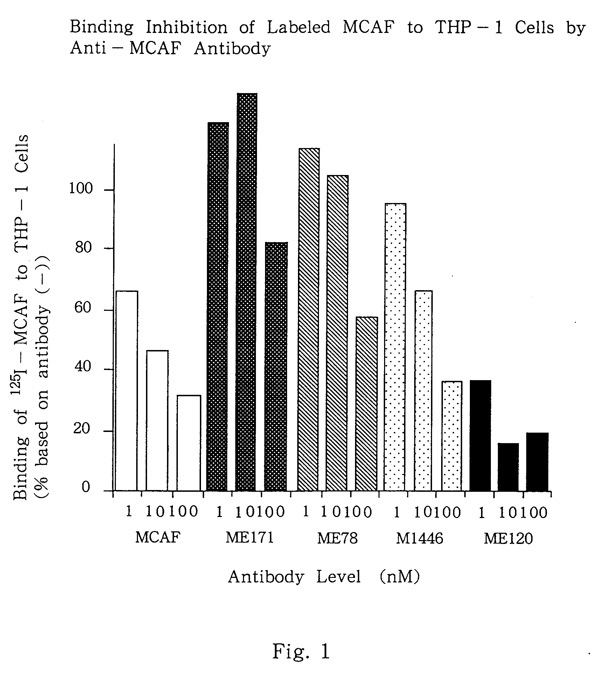

Fig. 1 shows binding inhibition of labeled MCAF to THP-1 cells by anti-MCAF antibody. The amount of ¹²⁵I-labeled MCAF is shown in terms of % based on the value in which the antibody was not added.

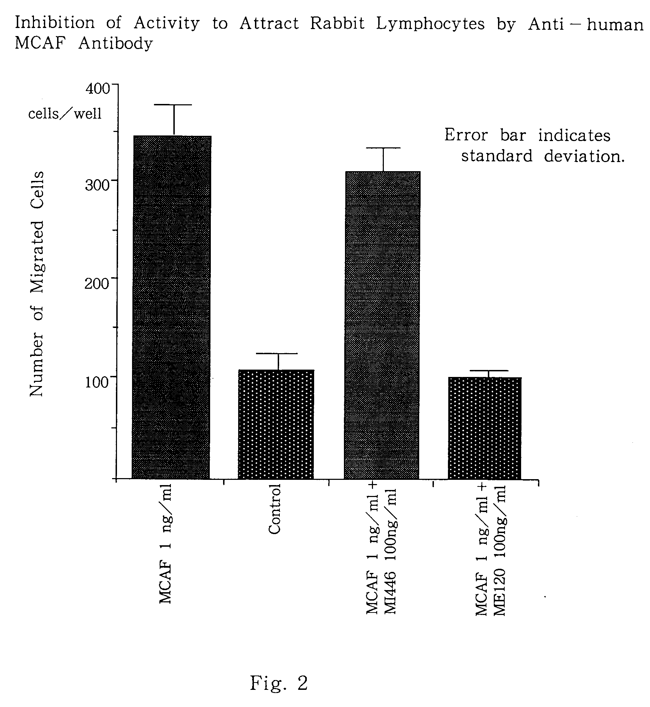

Fig. 2 shows inhibition effect by anti-human MCAF antibody to inhibit migration of rabbit lymphocytes. The first column shows the number of migrated cells when MCAF was added, the second column shows the number of migrated cells when nothing was added (control), the third column shows the number of migrated cells when MI446 antibody and MCAF were added, and the fourth column shows the number of migrated cells when ME120 antibody and MCAF were added.

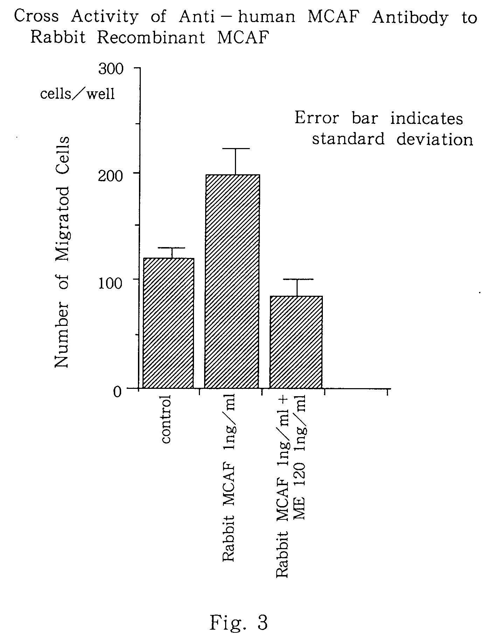

Fig. 3 shows the cross activity of anti-human MCAF antibody to rabbit recombinant MCAF. The first column shows the number of migrated cells in control, the second column shows the number of migrated cells when rabbit recombinant MCAF was added and the third column shows the number of migrated cells when rabbit recombinant MCAF and ME120 antibody were added.

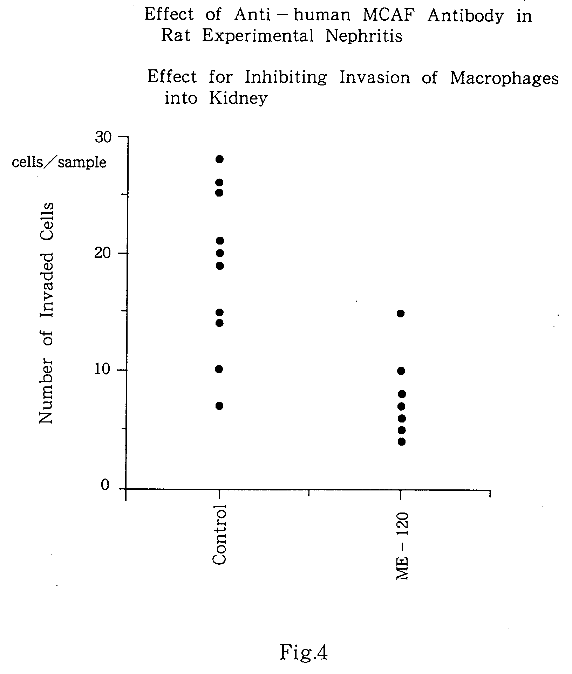

Fig. 4 shows effect of anti-human MCAF antibody in rat experimental nephritis, that is, the effect to inhibit invasion of macrophages into the kidney. On the left, the number of monocytes and macrophages in the microtubular space of frozen tissue sections of kidney of each animal to which PAN alone was administered is shown, and on the right, the number of monocytes and macrophages in the microtubular space of frozen tissue sections of kidney of each animal to which PAN and ME120 antibody were administered is shown.

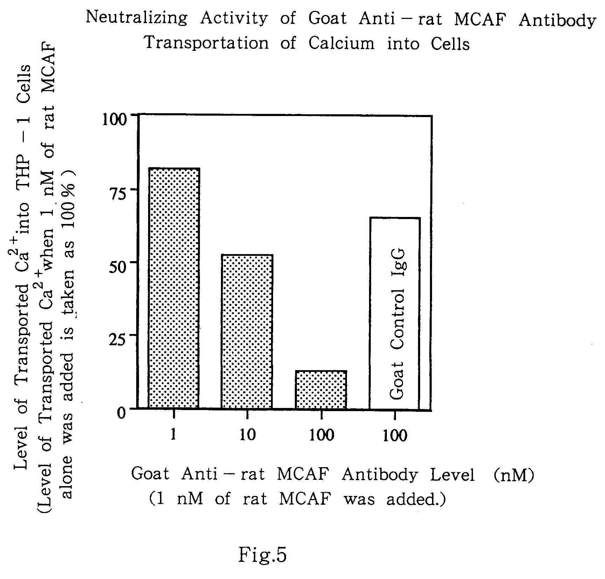

Fig. 5 shows the transportation of calcium ion into the cells observed after adding rat recombinant MCAF to THP-1 cells.

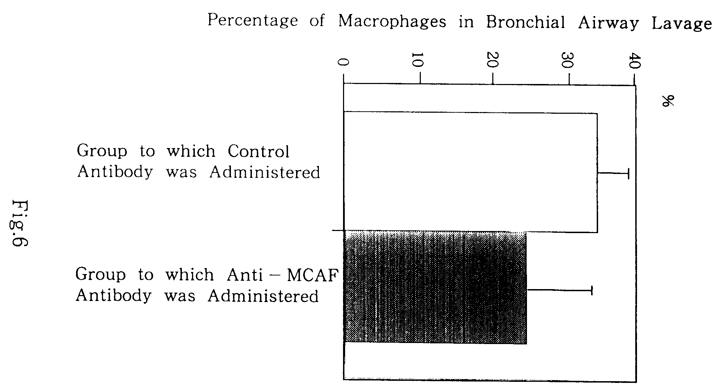

Fig. 6 shows the effect of anti-rat MCAF antibody on rat pneumonia model. Cytopreparations of the cells contained in rat bronchial airway lavage were made, the number of the cells and the number of macrophages were counted, and the percentage of the number of macrophages based on the total number of the cells contained in the lavage is shown.

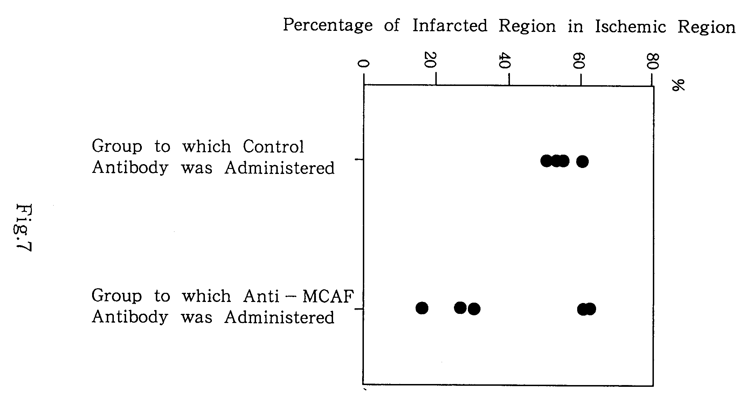

Fig. 7 shows the weight ratio of the infarcted region to the ischemic region of each rat. The ischemic region in the heart muscle was cut out and weighed after reperfusion operation after ischemic treatment, and the infarcted region in the ischemic region was taken out and weighed.

### BEST MODE FOR CARRYING OUT THE INVENTION

As mentioned above, the therapeutic agent according to the present invention comprises as an effective ingredient an antibody which inhibits migration of monocytes and/or macrophages to pathologic lesions. That is, the therapeutic agent according to the present invention comprises as an effective ingredient an antibody specific to a substance that gives chemotaxis of monocytes and/or macrophages to pathologic lesions (i.e., a substance that attracts monocytes or macrophages, hereinafter also referred to as "attracting substance" for short), which antibody can inhibit binding of the attracting substance with monocytes or macrophages.

A representative example of the attracting substance is MCAF. It has been reported that MCAF is produced *in vivo*, such as monocytes, blood cells such as macrophages and lymphocytes, as well as by various cells such as fibroblast cells, vascular endothelial cells, smooth muscle cells and various tumor cells, upon stimulation by IL-1, TNF, IFN-γ, LPS or the like.

MCAF is also called MCP-1 (monocyte chemoattractant protein-1) or GDCF (glioma-derived monocyte chemotactic factor). MCAF is a protein consisting of 76 amino acids, which has 4 cysteine residues. Identification of MCAF, MCP-1 and GDCF, as well as cloning of the gene, have been reported (K. Matsushima et al., J. Exp. Med., 169, 1485-1490, 1989; Y. Furutani et al., Biochem. Biophys. Res. Commun., 159, 249-255, 1989; E.R. Robinson et al., Proc. Natl. Acad. Sci. USA, 86, 1850-1854, 1989; and T. Yoshimura et al., FEBS Letters, 244, 487-493, 1989). Methods for producing and purufication of MCAF are known and also described in these references. In the description hereinbelow, "MCAF" is used as an abbreviation which also includes MCP-1 and GDCF.

MCAF is a member of CC chemokine family including members similar to each other in the number of amino acids including four cysteine residues. CC chemokine family is the collective name of physiologically active substances (chemokines) characterized by two juxtaposed cysteine residues and by having chemotaxis (N. Mukaida et al., Microbiol. Immunol. 36, 773-789, 1992). CC chemokine is also called "β chemokine" (Oppenheim et al., Annual Rev. Immunol., 9, 617, 1991). CC-chemokine family is a collective name of a group of low molecular weight proteins which have about 30% homology of the amino acid sequences and have the 4 cysteine residues at the corresponding sites. Known proteins other than MCAF, which belongs to this family includes structural analogues of MCAF such as RANTES, LD78, ACT2, I-309, MCP-2 and MCP-3 of human; and JE, MIP-1α, MIP-1β and TCA-3 of mouse. Among these, biologically active substances which make monocytes and/or macrophages migrate to the pathologic lesions include MCP-2 (E. Feifel et al., Immunogenetics 36, 104-109, 1992), MCP-3 (K.B.M. Reid, Immunol. Today 10, 177-180, 1989), RANTES (P.N. Barlow et al., J. Mol. Biol. 232, 268-284, 1993), JE (B.J. Rollins et al., Proc. Natl. Acad. Sci. U.S.A. 85, 3738-3742, 1988) and the like.

Although the description hereinbelow is directed to MCAF as an example, the same description is equally applied to the attracting substances other than MCAF.

The antibody employed in the present invention may be any antibody which is specific to an attracting substance such as the above-mentioned MCAF and which can inhibit binding of the attracting substance with monocytes or macrophages. The antibody may be a monoclonal antibody or a polyclonal antibody. The animal from which the antibody originates is not restricted and may be any animal such as human, mouse, rat, rabbit, dog, goat and the like. Further, as long as it can inhibit the binding between the attracting substance and the monocytes or macrophages, the antibody may be a modified antibody. For example, the modified antibody may be one in which a part of the sequence of the antibody is changed, substituted or deleted, or more than one sequence is inserted into the sequence of the antibody (e.g., binding fragments such as Fab fragment and F(ab')₂ fragment); a modified antibody artificially reconstituted so as to have a part of the sequence of human antibody (chimeric antibody and humanized antibody); or may be a modified antibody in which the antibody is bound to or mixed with various molecules such as polyethylene glycol. The term "antibody" in the claims of the present application includes these modifications of the antibody.

The neutralizing activity (i.e., inhibition activity against MCAF) of the antibody employed in the present invention may be confirmed by a competitive inhibition test of the action of MCAF. For example, the neutralizing activity may be confirmed by inhibition of migration of monocytes to MCAF. Alternatively, the neutralizing activity of the antibody may be quantified in terms of the degree of inhibition of the binding of MCAF to a human cell line THP1 originated from a leukemia patient. More particularly, in the binding test using isotope-labelled MCAF, the relationship between the molar ratio of the neutralizing antibody to MCAF and the inhibition ability may be employed as an index of the neutralizing activity. Any antibody may be employed in the present invention as long as it attains the purpose to effectively inhibit the action of MCAF *in vivo*. However, those antibodies which effectively inhibit the binding at a molar ratio to MCAF of, for example, 1-1000 times, more preferably 1-10 times, are used. The term "effectively inhibit" herein means that the inhibition rate is not less than 30%, preferably not less than 50%, more preferably not less than 80%.

For example, as described in Example 2 below, unlabeled MCAF *per se* had an inhibition rate of about 35% when the molar ratio to labeled MCAF was 1, and about 55% when the molar ratio was 10. A monoclonal antibody ME120 which is an embodiment of the present invention had an inhibition rate of 60% when the molar ratio was 1, and 85% when the molar ratio was 10. The term "inhibition rate" herein means the value obtained by subtracting the ratio (%) of bound labeled MCAF described in Example 2 from 100%.

In cases where the antibody used as the therapeutic agent of the present invention is a polyclonal antibody, the antibody may be obtained from antiserum prepared by immunizing a mammal with MCAF as an immunogen.

Monoclonal antibodies may be obtained as follows. That is, a mammal is immunized with MCAF as an immunogen and then antibody-producing cells such as spleen cells and lymphocytes obtained from the immunized animal are fused with myeloma cells to prepare hybridomas. Among the obtained hybridomas, the clones which produce antibodies that specifically react with MCAF are selected by ELISA or the like. To provide a therapeutic agent having high therapeutic activity, it is important to select at this stage an antibody whose affinity with the antigen is as high as possible. Then the obtained hybridoma is cultured and the monoclonal antibody can be purified from the culture supernatant. Alternatively, the hybridoma may be inoculated to an appropriate animal, and the monoclonal antibody can be purified from ascites of the animal. The monoclonal antibody may be purified by appropriate purification means which is a combination of, for example, chromatography employing a carrier having affinity to the antibody, ion-exchanging carrier, hydrophobic carrier and antibody-carrying carrier.

The obtained monoclonal antibodies do not necessarily have neutralizing activities of MCAF. Therefore, by the method as described in Example 2 below, their neutralizing activities are checked and those having neutralizing activities are selected.

The modified antibodies described above may be obtained by chemically modifying the obtained antibody. Alternatively, modified antibodies in which the amino acid sequence is changed, such as chimeric antibodies, may be obtained by culturing recombinant cells in which the gene encoding the modified antibody is introduced, by the method as described above, and recovering the modified antibody from the cultured cells. These methods have been established in this field.

The present invention first described that the monoclonal antibodies having neutralizing activities of MCAF are useful for therapy. Several cases where monoclonal antibodies specific to MCAF were produced have been reported (T. Yoshimura et al., J. Immunol., 147, 2229, 1991 and Japanese Laid-open Patent Application (Kokai) No. 5-276986). However, in any of these cases, it is not shown that a monoclonal antibody having the neutralizing activity is useful for actual therapy. Needless to say, no case has been reported in which the possibility to treat a diseased animal model is shown by administering the antibody to the animal. The present invention is the first case which showed that a monoclonal antibody having an activity to neutralize MCAF actually cures diseased animal models. As mentioned above, even if an antibody which reacts with MCAF is obtained, the antibody does not necessarily have the neutralizing activity, and it cannot be known for which disease the antibody is effective.

The present inventors discovered that the 4 anti MCP-1 monoclonal antibodies E11, B8, A3 and F9, described in the above-mentioned T. Yoshimura et al., J. Immunol., 147, 2229, 1991 as well as the anti MCP-1 monoclonal antibodies 1, 2, 3 and 4 described in Japanese Laid-open Patent Application (Kokai) No. 5-276986 are also useful as the effective ingredient of the therapeutic agent according to the present invention.

The present inventors actually administered these antibodies *in vivo* and showed that these antibodies are useful as therapeutic agents, thereby completing the present invention. Thus, the antibody employed in the present invention has activity to neutralize the action of MCAF. Therefore, upon administering the antibody employed in the present invention *in vivo*, the antibody neutralizes MCAF *in vivo* and cures the diseases caused by MCAF. The diseases which may be cured by the therapeutic agent of the present invention may include all diseases related to MCAF.

Examples of such diseases include cardiovascular diseases, inflammation, allergic diseases, nephropathy and metastasis of malignant neoplasm. More specifically, examples of such diseases include nephritis, glomerulotubular nephritis, pyelitis, lung fibrosis, pneumonia, ulcerative colitis, chronic rheumatoid arthritis, systemic erythematodes, gout, bronchial asthma, atopic dermatitis, Crohn's disease, arterial sclerosis, re-occlusion of coronary artery after surgery including PTCA, myocardial infarction, metastasis of epithelial cancer and metastasis of malignant sarcoma.

The therapeutic agent of the present invention may contain as an effective ingredient the above-mentioned antibody alone or may also contain pharmaceutically acceptable carriers or additives. Examples of the pharmaceutically acceptable carriers and additives include water, pharmaceutically acceptable organic solvents, collagen, polyvinyl alcohol, polyvinylpyrrolidone, carboxyvinyl polymer, sodium carboxymethyl cellulose, sodium polyacrylate, sodium alginate, water-soluble dextran, sodium carboxymethyl starch, pectin, methyl cellulose, ethyl cellulose, xanthane gum, gum arabi, casein, gelatin, agar, polyethylene glycol, diglycerin, glycerin, propylene glycol, polyethylene glycol, vaseline, paraffin, stearyl alcohol, stearic acid, human serum albumin (HSA), mannitol, sorbitol, lactose and pharmaceutically acceptable surfactants. The additives may be appropriately selected depending on the formulation of the therapeutic agent of the present invention from the above-mentioned additives and combinations thereof, but, needless to say, the additives are not restricted to those mentioned above.

According to the present invention, other drugs, biological formulations and synthetic pharmaceuticals may be simultaneously or sequentially administered together with MCAF. The other drugs may be selected from anti-inflammatory agents, anti-allergy agents, thrombus formation-inhibiting agents, anti-tumor agents, anti-hyperlipidemia agents and other drugs which reinforce or supplement the effects of the present invention.

The administration route is not restricted. Depending on the purpose of administration, the therapeutic agent may be administered topically or systemically. External formulations include application formulations such as ointments, gels, creams, emulsions and lotions; pasting formulations such as tapes and patches; spray; and powder. Formulations for systemic administration include injection solutions and sustained release preparations employing various carriers.

Administration dose of the effective ingredient may be selected from the range of 0.001 mg to 1000 mg per 1 kg of body weight per day, preferably 0.01 mg to 10 mg per body weight per day. However, the administration dose varies depending on symptom and so the administration dose is not restricted to the range mentioned above. The times of administration may be one to several times per day, or once per two or several days, although not restricted thereto.

A specific example of a formulation which is used as an injection solution is described in the following.

| | |
|---|---|
| Antibody | 50 mg |
| Phosphate Buffer | 10 ml |

The present invention will now be described in more concretely by way of examples. It should be noted that the examples are presented for the illustration purpose only and should not be interpreted in any restrictive way.

### Example 1 Preparation of Monoclonal Antibodies

Monoclonal antibodies described below were prepared by the method described in Japanese Laid-open Patent Application (Kokai) No. 6-205690. That is, natural type MCAF sample purified from culture supernatant of human normal fibroblast cells was used as an immunogen. Immunization was carried out by intraperitoneally administering the MCAF to five female Balb/c mice of 8 weeks old. The first immunization was carried out by administering a mixture of the MCAF and potassium aluminum sulfate adjuvant to which dead *Bordetella pertussis* cells are added. The second and subsequent immunizations were carried out by administering a mixture of MCAF and potassium aluminum sulfate adjuvant or by administering MCAF solution in PBS. Three days after the final immunization, spleen was removed from each mouse and the spleen cells were fused with mouse myeloma cells P3U1 (P3X63-Ag8U1). The cells after the cell fusion treatment were suspended in RPMI1640 medium containing 15% fetal calf serum (commercially available from Biocell), 2 ng/ml of human IL-6 (purified from culture supernatant of human normal fibroblast cells) and HAT (commercially available from Boehringer). The cell suspension was dividedly placed in wells of a 96-well microplate at a cell density of 2 - 5 x 10⁵ cells/200 µl/well, and the cells were cultured under 7% CO₂ at 37°C.

Screening of the antibody-producing cells was carried out by ELISA for detecting binding of the antibody to immobilized MCAF. In each well of a 96-well microplate (commercially available from Nunc), 0.5 µg/ml of MCAF solution in PBS was placed and the solution was incubated overnight at 4°C so as to immobilize MCAF. After carrying out blocking with 0.5% bovine serum albumin (BSA) solution in PBS, 100 µl of supernatant of the HAT medium containing the hybridomas (9-10 days after cell fusion) was allowed to react with the immobilized MCAF at room temperature for 1 hour, and then HRP-labeled goat anti-mouse IgG antibody (commercially available from Cappel) was allowed to react therewith at room temperature for 30 minutes. At the intervals between each reaction mentioned above, the wells were washed three times in each interval with PBS containing 0.05% Tween 20 so as to remove non-reacted reagents. Finally, substrate of the enzyme (0.1 M sodium citrate acetate buffer, pH5.5, containing 0.006% hydrogen peroxide and 0.2 mg/ml of 3,3',5,5'-tetramethylbenzidine (TMB)) was added to carry out coloring reaction. Positive wells were identified based on the generated color. To each of the positive wells, 100 µl of HT medium was added and the obtained mixture was incubated overnight. The resultant was 3-fold diluted in serial fashion and ELISA was carried out again. Clones with which coloring was observed at a high fold dilution were selected and cloned by limiting dilution method. The above-mentioned screening and cloning steps were repeated twice and 7 monoclonal hybridomas were established. The names and properties of the monoclonal antibodies obtained from these 7 hybridomas are summarized in Table 1. Among these, both ME120 and MI446 belong to the subclass IgG1_{K}. The hybridomas producing these monoclonal antibodies were deposited in National Institute of Bioscience and Human-Technology, Agency of Industrial Science and Technology at 1-3, Higashi 1-chome, Tsukuba-shi, Ibaraki-ken, 305 Japan on November 18, 1992 under accession Nos. FERM P-13293 and FERM P-13294, respectively, and the deposition was converted to international deposition under the Budapest Treaty on April 6, 1995, under accession Nos. FERM BP-5067 and FERM BP-5068, respectively. Both of these monoclonal antibodies bound to MCAF transferred to a membrane in Western blot analysis after electrophoresis.

**Table 1**

| Analysis of Antibodies | | | | |
|---|---|---|---|---|
| Clone | Cell Fusion Method | Subclass | Western Blot | |
| | | | 9 kD | 13 kD |
| MB171 | PEG | IgG1·_{K} | + | + |
| ME61 | Electrical Cell Fusion | IgG1·_{K} | + | + |
| ME78 | Electrical Cell Fusion | IgG1·_{K} | + | + |
| ME120 | Electrical Cell Fusion | IgG1·_{K} | + | + |
| ME190 | Electrical Cell Fusion | IgG1·_{K} | + | + |
| ME198 | Electrical Cell Fusion | IgG1·_{K} | + | + |
| MI446 | Electrical Cell Fusion | IgG1·_{K} | + | + |

### Example 2 Selection of Neutralizing Antibody

By checking the inhibition of binding of MCAF to receptors, an antibody having the neutralizing activity was discovered. As the cells expressing the receptors, 10⁶ cells/250 µl of THP-1 cells (Int. J. Cancer 261, 171, 1980; ATCC TIB202) were used. To this cell suspension, ¹²⁵I-labeled MCP-1 was added to a final concentration of 1 nM and the mixture was left to stand at 37°C for 30 minutes. The obtained mixture was then overlaid on 150 µl of 2/3 mixture of dioctylphthalic acid/dibutylphthalic acid and the resultant was centrifuged (5000 rpm, 2 minutes) and cell fraction was collected, followed by measurement of bound ¹²⁵I-labeled MCAF with a gamma counter. Taking the amount of the bound ¹²⁵I-labeled MCAF as 100%, the percentages of the amounts of the bound ¹²⁵I-labeled MCAF in the presence of 1 nM, 10 nM and 100 nM (1, 10 and 100 times concentration of ¹²⁵I-labeled MCAF) of the anti-MCAF mouse monoclonal antibody (MB171, ME78, ME120, ME190, ME198 or MI446), respectively, are shown. When ME120 was added to 1 nM and 10 nM, the amount of the bound labeled MCAF was about 37% and 16%, respectively, based on that obtained when this antibody was not added. When unlabeled MCAF was added to 1 nM, 10 nM and 100 nM, the obtained values were 64%, 45% and 32%, respectively. Thus, ME120 has a not less than 10 times higher activity to inhibit the binding than unlabeled MCAF.

### Example 3 Confirmation of Inhibition by Neutralizing Antibody of Migration and Activation of Monocytes and Macrophages by MCAF

Peripheral blood from a rabbit (New Zealand White, purchased from SLC) was placed on a separation solution having a specific density of 1.077 g/ml and lymphocyte fraction was recovered. The obtained lymphocytes were suspended in RPMI1640 medium containing 0.1% BSA to a population density of 5 x 10⁵ cells/ml to prepare a suspension of rabbit lymphocytes. 100 ng/ml of anti-human MCAF antibody and 1 ng/ml of human MCAF were preliminarily reacted at 37°C for 20 minutes. This antibody/MCAF mixture was placed in the lower chamber of each of 48-well chemotactic chambers. On each of the lower chambers, a polycarbonate membrane was placed and an upper chamber of chemotactic chamber was set on the membrane. The suspension of rabbit lymphocytes was placed in each of the upper chambers and incubated at 37°C for 90 minutes under 5% CO₂. Thereafter, the migrated cells, together with the membrane, were subjected to Giemsa staining and the number of the migrated cells was counted. The results are shown in Fig. 2. As shown, 100 ng/ml of the neutralizing antibody ME120 completely inhibited the attracting activity of 1 ng/ml of MCAF. This inhibition activity is 10 times that of MCAF in molar ratio. However, no inhibition activity was observed with MI446 at the same concentration. Further, activity for inhibiting activation of monocytes and macrophages by MCAF was tested. By adding MCAF to THP-1 cells, transportation of calcium into the cells is observed as a signal of activation of the cells. It was confirmed that this antibody inhibits the transportation of calcium into the cells, which transportation is induced by MCAF.

### Example 4 Activity of Anti-human MCAF Antibody to Inhibit Migration of Monocytes and Macrophages Induced by Animal MCAF

Peripheral blood from a rabbit (New Zealand White, purchased from SLC) was placed on a separation solution having a specific density of 1.077 g/ml and lymphocyte fraction was recovered. The obtained lymphocytes were suspended in RPMI1640 medium containing 0.1% BSA to a population density of 5 x 10⁵ cells/ml to prepare a suspension of rabbit lymphocytes. Anti-human MCAF antibody ME-120 and rabbit recombinant MCAF (obtained by expressing rabbit cDNA in monkey COS 1 cells, which cDNA is inserted in pSRα expression vector for animal cells) were preliminarily incubated at 37°C for 20 minutes. This antibody/MCAF mixture was placed in the lower chamber of each of 48-well chemotactic chambers. On each of the lower chambers, a polycarbonate membrane was placed and an upper chamber of chemotactic chamber was set on the membrane. The suspension of rabbit lymphocytes was placed in each of the upper chambers and incubated at 37°C for 90 minutes under 5% CO₂. Thereafter, the migrated cells, together with the membrane, were subjected to Giemsa staining and the number of the migrated cells was counted. As shown in Fig. 3, 1 ng/ml of the anti-human MCAF antibody ME120 inhibited the activity of 1 ng/ml of rabbit recombinant MCAF to attract rabbit peripheral blood lymphocytes.

### Example 5 Effect of Anti-human MCAF Antibody on Rat Experimental Nephritis

Puromycin Aminonucleoside (hereinafter referred to as "PAN") was administered to rats (SD rat, purchased from Charles River) via tail vein with a dose of 10 mg/100 g body weight, the resulting rats being used as nephritis models. Simultaneously with the administration of PAN, the antibody ME120 was administered via tail vein with a dose of 10 mg/kg body weight. On the day 5 and day 10, the same amount of ME120 antibody was further administered. To the control group, the same volume of physiological saline was administered. Two weeks after the administration of PAN, sections of frozen kidney tissues were prepared and the numbers of monocytes and macrophages in the microtubular space were compared. The results are shown in Fig. 4. Although prominent invasion of monocytes and macrophages into the microtubular space was induced by PAN administration, the numbers in the group to which anti-human MCAF antibody was administered (10 mg/kg) were significantly reduced when compared with the numbers in the control group.

### Example 6 Effect of Goat Anti-rat Polyclonal Antibody in Rat Experimental Pneumonia

To each rat (SD rat, purchased from Charles River), 5 mg of glucan was administered via tail vein, thereby preparing pneumonia models. Seventy two hours after the administration of glucan, preparations of the cells in bronchial airway lavage was prepared, and the number of cells invaded into the lung and the number of macrophages among the invaded cells were counted under microscope after staining the cells. Counting of the cells was always carried out under blind. Goat anti-rat recombinant MCAF polyclonal antibody was prepared by immunizing a goat with the rat recombinant MCAF (obtained from Cancer Research of Kanazawa University) three times at 2 weeks intervals together with Freund's complete adjuvant, collecting the total blood at 2 weeks after the final immunization, and purifying IgG fraction by subjecting the serum fraction to ammonium sulfate precipitation, ion-exchange chromatography and protein A affinity chromatography. The activity of this polyclonal antibody to activate macrophages was checked as in Example 3 using THP-1 cells (Fig. 5). As shown, it was confirmed that this polyclonal antibody inhibited activation of the cells at a level 100 times that of the rat recombinant MCAF. The antibody was administered twice at 24 hours and 48 hours after the administration of glucan. For the control group, IgG purified from normal goat serum was administered in the same manner. The dose of the anti-rat MCAF antibody and the does of the purified IgG were both 100 mg/kg.

As shown in Fig. 6, invasion of macrophages into the lung was inhibited in the group to which the anti-MCAF antibody was administered.

### Example 7 Effect of Goat Anti-rat MCAF Polyclonal Antibody on Rat Experimental Myocardial Infarction

Rats (Wister rat, body weight 250-300 g) were anesthetized with pentobarbital and the running portion of left coronary of each rat was ligated after thoracotomy. After one hour's ischemic time, the heart was reperfused. After the reperfusion, goat anti-rat MCAF polyclonal antibody was administered via tail vein at a dose of 10 mg/kg. To the control group, goat IgG was administered in the same manner. Twenty four hours after the administration, non-ischemic region was stained with Evans' Blue and the ischemic region was cut out. The ischemic region was stained with 1% TTC at 37°C for 5 minutes. The infarcted region was cut out and its tissue wet weight was measured.

The evaluations are shown in terms of the percentage of the infarcted region based on the ischemic region. As shown in Fig. 7, in the control group, infarcted region as much as 60% of the ischemic region was observed in all of the 5 animals. In contrast, in the group to which the anti-MCAF antibody was administered, about the same percentage of the infarcted region as in the control group was observed in only 2 animals among the 5 animals. In the remaining 3 animals, the percentage of the infarcted region was only 18-30%.

## Claims

1. A therapeutic agent for diseases characterized by abnormal accumulation or activation of leukocytes, comprising as an effective ingredient an antibody which inhibits migration of monocytes and/or macrophages to pathologic lesions.

2. The therapeutic agent according to claim 1, wherein said antibody is an anti-monocyte chemotactic and activating factor antibody.

3. The therapeutic agent according to claim 2, wherein said anti-monocyte chemotactic and activating factor antibody is an anti-human monocyte chemotactic and activating factor antibody.

4. The therapeutic agent according to claim 2 or 3, wherein said anti-monocyte chemotactic and activating factor antibody inhibits, at an amount of not more than 10 times by mole of the monocyte chemotactic and activating factor, not less than 30% of monocyte-attracting activity of the monocyte chemotactic and activating factor.

5. The therapeutic agent according to claim 4, wherein said anti-monocyte chemotactic and activating factor antibody inhibits, at an amount of not more than 10 times by mole of the monocyte chemotactic and activating factor, not less than 50% of monocyte-attracting activity of the monocyte chemotactic and activating factor.

6. The therapeutic agent according to claim 5, wherein said anti-monocyte chemotactic and activating factor antibody inhibits, at an amount of not more than 10 times by mole of the monocyte chemotactic and activating factor, not less than 80% of monocyte-attracting activity of the monocyte chemotactic and activating factor.

7. The therapeutic agent according to claim 5, wherein said anti-monocyte chemotactic and activating factor antibody is monoclonal antibody ME120 (FERM BP-5067).

8. The therapeutic agent according to any one of claims 1-7, wherein said diseases characterized by abnormal accumulation or activation of leukocytes are selected from the group consisting of cardiovascular diseases, inflammation, allergic diseases, autoimmune diseases, nephropathy and metastasis of malignant neoplasm.

9. The therapeutic agent according to any one of claims 1-7, wherein said diseases characterized by abnormal accumulation or activation of leukocytes are selected from the group consisting of arterial sclerosis, re-occlusion of coronary artery after surgery including PTCA, myocardial infarction, ulcerative colitis, chronic rheumatoid arthritis, Crohn's disease, atopic dermatitis, lung fibrosis, pneumonia, nephritis, glomerulotubular nephritis, pyelitis, metastasis of epithelial cancer and metastasis of malignant sarcoma.

10. The therapeutic agent according to claim 9, wherein said diseases characterized by abnormal accumulation or activation of leukocytes are selected from the group consisting of nephritis, pneumonia and myocardial infarction.
